# EUROPEAN PATENT APPLICATION

(11) **EP 4 082 521 A1**
(43) Date of publication of application: **02.11.2022**
(21) Application number: 20906968.1
(22) Date of filing: 15.12.2020
(51) Int. Cl.: A61K 8/898, A61K 8/34, A61K 8/37, A61K 8/41, A61K 8/81, A61K 8/86, A61K 8/891, A61Q 5/00, A61Q 5/10

(54) **FIBER TREATMENT AGENT COMPOSITION**

(30) Priority: 23.12.2019 JP 2019232110
(71) Applicant: Kao Corporation, Chuo-ku Tokyo 103-8210 (JP)
(72) Inventor: MURAKI, Manami, Tokyo 131-8501 (JP)
(74) Representative: Hoffmann Eitle
(86) International application number: PCT/JP2020/046802
(87) International publication number: WO 2021/131915

(57) **Abstract**

A fiber treatment agent composition comprising the following components (A) and (B), wherein a content of the component (A) is 0.05% by mass or more and 5.0% by mass or less, the content of the component (B) is 0.05% by mass or more and 10.0% by mass or less, and a mass ratio of the component (B) to the component (A), (B)/(A), is 150 or less: (A) a self-crosslinking compound; and (B) one or more color deepening agents selected from the group consisting of a nonvolatile silicone, a polyhydric alcohol polymer, a fatty acid ester, and an acrylic resin (excluding agents corresponding to the component (A)).

## Description

### Technical Field

The present invention relates to a fiber treatment agent composition.

### Background Art

In recent years, hair ends, in particular, are being more seriously damaged due to chemical treatments such as hair coloring and perms as well as hair setting or the like by using heat from hair irons, hair dryers and the like, which is becoming more common mainly among young women. Hair damage causes hydrophilization of the hair surface and increased surface friction, resulting not only in problems such as tangling of the hair ends during styling and poor finger combability, but also in a problem that because cavities are formed inside the hair, the scattered reflection of light causes the appearance of the hair to look whitish, resulting in an unhealthy look.

As a technique for improving the color of damaged hair, Patent Literature 1 discloses that hair dulled by damage can be vividly shown in its original color and its color saturation can be enhanced by washing and conditioning the hair with a shampoo containing a water-insoluble modified silicone containing an amino group or a quaternary ammonium group.

(Patent Literature 1) JP-A-2006-206585

### Summary of the Invention

The present invention provides a fiber treatment agent composition comprising the following components (A) and (B), wherein a content of the component (A) is 0.05% by mass or more and 5.0% by mass or less, a content of the component (B) is 0.05% by mass or more and 10.0% by mass or less, and a mass ratio of the component (B) to the component (A), (B)/(A), is 150 or less:
(A) a self-crosslinking compound; and
(B) one or more color deepening agents selected from the group consisting of a nonvolatile silicone, a polyhydric alcohol polymer, a fatty acid ester, and an acrylic resin (excluding agents corresponding to the component (A)).

Further, the present invention provides a fiber treatment method comprising applying the above-described fiber treatment agent composition to a fiber surface, and then drying without rinsing off.

In addition, the present invention provides a fiber treatment method comprising applying a composition containing the following component (A) to a fiber surface, then, without drying, applying a composition containing the following component (B) to the applied portion, and then drying without rinsing off:
(A) a self-crosslinking compound; and
(B) one or more color deepening agents selected from the group consisting of a nonvolatile silicone, a polyhydric alcohol polymer, a fatty acid ester, and an acrylic resin (excluding agents corresponding to the component (A)).

Still further, the present invention provides a fiber treatment method comprising applying a composition containing the following component (B) to a fiber surface, then, without drying, applying a composition containing the following component (A) to the applied portion, and then drying without rinsing off:
(A) a self-crosslinking compound; and
(B) one or more color deepening agents selected from the group consisting of a nonvolatile silicone, a polyhydric alcohol polymer, a fatty acid ester, and an acrylic resin (excluding agents corresponding to the component (A)).

### Detailed Description of the Invention

The technique described in Patent Literature 1 does not sufficiently improve color saturation, and is not satisfactory as a technique for improving the appearance of damaged hair.

Therefore, the present invention relates to a fiber treatment agent composition which, by treating fibers such as hair, can make the color of the fiber surface appear deeper and can maintain this effect even when the hair is repeatedly washed after treatment.

As a result of intensive investigations, the present inventors found that the above-described problems can be solved by using a component capable of increasing the thickness of a film formed on a fiber surface together with a specific self-crosslinking compound, and completed the present invention.

The fiber treatment agent composition of the present invention can make the surface of a fiber, such as hair, look a deeper color.

### [Component (A): Self-crosslinking compound]

Examples of the self-crosslinking compound of the component (A) include the following components (A1) to (A3):
(A1) an alkoxysilyl group-containing silicone;
(A2) an alkoxysilyl group-containing acrylate compound; and
(A3) an alkoxysilyl group-containing alkylamine.

The components (A1) to (A3) all have an alkoxysilyl group - SiR¹ₙ(OR²)₃₋ₙ.

In the formula, R¹ and R² independently represent a monovalent hydrocarbon group, and n represents a number from 0 to 2.

The alkoxysilyl group-containing silicone of the component (A1) has an alkoxysilyl group bonded to an organopolysiloxane residue.

In this case, the organopolysiloxane residue contains an amino group, and more preferably an amino group and a polyether group. Specifically, examples of the component (A1) include the epoxyaminosilane copolymer and (amodimethicone/morpholinomethyl silsesquioxane) copolymer shown below, and an epoxyaminosilane copolymer is preferable.

The epoxyaminosilane copolymer is a reaction product of the following compounds (a) to (d):
(a) a polysiloxane having at least two oxiranyl groups or oxetanyl groups;
(b) a polyether having at least two oxiranyl groups or oxetanyl groups;
(c) aminopropyltrialkoxysilane; and
(d) a compound selected from the group consisting of the following primary and secondary amines:
   primary amines: methylamine, ethylamine, propyleneamine, ethanolamine, isopropylamine, butylamine, isobutylamine, hexylamine, dodecylamine, oleylamine, aniline, aminopropyltrimethylsilane, aminopropyltriethylsilane, aminomorpholine, aminopropyldiethylamine, benzylamine, naphthylamine, 3-amino-9-ethylcarbazole, 1-aminoheptafluorohexane, and 2,2,3,3,4,4,5,5,6,6,7,7,8,8,8-pentadecafluoro-1-octaneamine; and
   secondary amines: methylethylamine, methyloctadecylamine, diethanolamine, dibenzylamine, dihexylaminedicyclohexylamine, piperidine, pyrrolidinephthalimide, and polymer amines.

### <Compounds (a) and (b)>

The compound (a) is a polysiloxane containing at least two oxiranyl groups or oxetanyl groups. Examples of the compound (a) include compounds of the following formula (1).

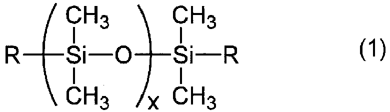

In the formula, R represents a hydrocarbon group which has an oxiranyl group or oxetanyl group at an end, optionally contains a hetero atom, and has 1 to 6 carbon atoms, and x represents a number from 1 to 1000.

The compound (b) is a polyether containing at least two oxiranyl groups or oxetanyl groups. Examples contains groups of the following formula (2).

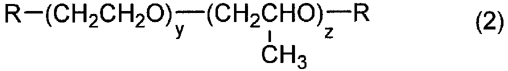

In the formula, R has the same meaning as described above, y represents a number from 1 to 100, z represents a number from 0 to 100, and y + z is a number from 1 to 200.

In formulas (1) and (2), the heteroatom optionally included by R is preferably an oxygen atom. Examples of R include an oxiranylmethyl group (glycidyl group), an oxiranylmethoxy group (glycidyloxy group), an oxiranylmethoxypropyl group (glycidyloxypropyl group), an oxetanylmethyl group, an oxetanylmethoxy group, an oxetanylmethoxypropyl group, a 3-ethyloxetanylmethyl group, and the like. Among them, a hydrocarbon group that has an oxiranyl group, optionally has a hetero oxygen atom, and has 1 to 4 carbon atoms is preferable, and at least one selected from the group consisting of an oxiranylmethyl group (glycidyl group), an oxiranylmethoxy group (glycidyloxy group), and an oxiranylmethoxypropyl group (glycidyloxypropyl group) is more preferable.

### <Compound (c)>

The compound (c) is an aminopropyltrialkoxysilane. Examples of the alkoxy group in the compound (c) include alkoxy groups having 1 to 6 carbon atoms, preferably 2 to 4 carbon atoms, further preferably 3 carbon atoms, and among which an isopropoxy group is preferable. Examples of the compound (c) include aminopropyltrimethoxysilane, aminopropyltriethoxysilane, aminopropyltripropoxysilane, aminopropyltriisopropoxysilane, aminopropyltributoxysilane, and aminopropyltri-tert-butoxysilane. Among them, aminopropyltriisopropoxysilane is preferable. The compound (c) can be used alone or in combination of two or more.

### <Compound (d)>

The compound (d) is a compound selected from the group consisting of the following primary and secondary amines:
primary amines: methylamine, ethylamine, propyleneamine, ethanolamine, isopropylamine, butylamine, isobutylamine, hexylamine, dodecylamine, oleylamine, aniline, aminopropyltrimethylsilane, aminopropyltriethylsilane, aminomorpholine, aminoethyldimethylamine, aminoethyldiethylamine, aminoethyldibutylamine, aminopropyldimethylamine, aminopropyldiethylamine, aminopropyldibutylamine, benzylamine, naphthylamine, 3-amino-9-ethylcarbazole, 1-aminoheptafluorohexane, and 2,2,3,3,4,4,5,5,6,6,7,7,8,8,8-pentadecafluoro-1-octaneamine; and
secondary amines: methylethylamine, methyloctadecylamine, diethanolamine, dibenzylamine, dihexylamine, dicyclohexylamine, piperidine, pyrrolidinephthalimide, and polymer amines.

Among these, a primary amine is preferable, and at least one selected from the group consisting of aminopropyldiethylamine, aminopropyldimethylamine, and aminopropyldibutylamine is more preferable. The compound (d) can be used alone or in combination of two or more.

The reaction of the compounds (a) to (d) is performed for a certain time under reflux in a solvent such as isopropanol. The molar ratio of the oxiranyl groups or the oxetanyl groups in the compound (a) and (b) to the amino group in the compound (c) is preferably 1 or more, more preferably 1.1 or more, and further preferably 1.2 or more, and preferably 4 or less, more preferably 3.9 or less, and further preferably 3.8 or less.

Examples of the epoxyaminosilane copolymer among the component (A) include Silsoft CLX-E having the INCI name polysilicone-29 (active amount of 15% by mass, including dipropylene glycol and water, manufactured by Momentive Performance Materials). Examples of the (amodimethicone/morpholinomethyl silsesquioxane) copolymer among the component (A1) include Belsil ADM 6300 and 8301 (manufactured by Wacker Asahikasei).

The alkoxysilyl group-containing acrylate compound of the component (A2) is a compound in which an alkoxysilyl group is bonded to a group of the following formula (3). 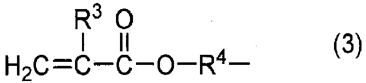 wherein R³ represents a hydrogen atom or a methyl group, and R⁴ represents a divalent saturated hydrocarbon group having 1 to 6 carbon atoms.

R⁴ in formula (3) is preferably a group having 2 to 4 carbon atoms, and among them a trimethylene group is preferable. Examples of the component (A2) include silane coupling agents such as 3-methacryloxypropylmethyldimethoxysilane (KBM-502, manufactured by Shin-Etsu Chemical Co., Ltd.), 3-methacryloxypropyltrimethoxysilane (KBM-503, manufactured by Shin-Etsu Chemical Co., Ltd.), 3-methacryloxypropylmethyldiethoxysilane (KBE-502, manufactured by Shin-Etsu Chemical Co., Ltd.), 3-methacryloxypropyltriethoxysilane (KBE-503, manufactured by Shin-Etsu Chemical Co., Ltd.) and 3-acryloxypropyltrimethoxysilane (KBM-5103, manufactured by Shin-Etsu Chemical Co., Ltd.).

The alkoxysilyl group-containing alkylamine of the component (A3) is a compound in which the alkoxysilyl group is bonded to a group of the following formula (4).

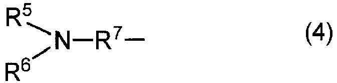

In the formula, R⁵ and R⁶ represent a hydrogen atom or a hydrocarbon group optionally substituted with an amino group, or R⁵ and R⁶ jointly form an alkylidene group, and R⁷ represents a divalent hydrocarbon group having 1 to 6 carbon atoms.

R⁷ in formula (4) preferably has 2 to 4 carbon atoms, and of those, a trimethylene group is preferred. Examples of the component (A3) include N-2-(aminoethyl)-3-aminopropylmethyldimethoxysilane (KBM-602, manufactured by Shin-Etsu Chemical Co., Ltd.), N-2-(aminoethyl)-3-aminopropyltrimethoxysilane (KBM-603, manufactured by Shin-Etsu Chemical Co., Ltd.), 3-aminopropyltriethoxysilane (KBM-903, manufactured by Shin-Etsu Chemical Co., Ltd.), 3-aminopropyltriethoxysilane (KBE-903, manufactured by Shin-Etsu Chemical Co., Ltd.), 3-triethoxysilyl-N-(1,3-dimethylbutylidene)propylamine (KBE-9103P, manufactured by Shin-Etsu Chemical Co., Ltd.), N-phenyl-3-aminopropyltrimethoxysilane hydrochloride (KBM-573, manufactured by Shin-Etsu Chemical Co., Ltd.), N-(vinylbenzyl)-2-aminoethyl-3-aminopropyltrimethoxysilane (KBM-575, manufactured by Shin-Etsu Chemical Co., Ltd.), and the like. Of those, 3-aminopropyl-triethoxysilane, 3-aminopropylmethyldiethoxysilane, N-(2-aminoethyl)-3-aminopropyltriethoxysilane, 3-(2-aminoethylamino)propylmethyldiethoxysilane, and the like are preferred.

The content of the component (A) in the fiber treatment agent composition of the present invention is, from the viewpoint of excellent film formability and the accompanying expression of a deeper color of the fiber surface, 0.05% by mass or more, preferably 0.1% by mass or more, more preferably 0.2% by mass or more, and further preferably 0.25% by mass or more, and 5.0% by mass or less, preferably 3.0% by mass or less, more preferably 1.0% by mass or less, and further preferably 0.5% by mass or less.

### [Component (B): Color deepening agent]

The component (B) is one or more color deepening agents selected from the group consisting of a nonvolatile silicone, a polyhydric alcohol polymer, a fatty acid ester, and an acrylic resin. However, agents corresponding to the component (A), that is, self-crosslinking compounds, are excluded from the component (B). The color deepening agent of the component (B) is a component that can have an effect of making the color of the fiber surface appear darker by increasing the thickness of the film formed on the fiber surface.

Examples of the nonvolatile silicone include dimethylpolysiloxane, amino-modified polysiloxane, phenyl-modified polysiloxane, polyglycerin-modified polysiloxane, silicone resin, acrylic-modified polysiloxane, alkyl-modified polysiloxane, methacryl-modified polysiloxane, carboxyl-modified polysiloxane, aminopolyether-modified polysiloxane, oxazoline-modified polysiloxane, polyether-modified polysiloxane, fluorine-modified polysiloxane, mercapto-modified polysiloxane, hydrogen-modified polysiloxane, aralkyl-modified polysiloxane, epoxy-modified polysiloxane, hydroxy-modified polysiloxane, and the like. Among them, oxazoline-modified polysiloxane is preferable, and polysilicone-9 is more preferable.

Examples of the polyhydric alcohol polymer include diethylene glycol, dipropylene glycol, triethylene glycol, polypropylene glycol, tetraethylene glycol, diglycerin, polyethylene glycol, triglycerin, tetraglycerin, polyglycerin, and the like.

Examples of the fatty acid ester include lauric acid esters, myristic acid esters, palmitic acid esters, oleic acid esters, phthalic acid esters, adipic acid esters, caprylic acid esters, capric acid esters, behenic acid esters, methacrylic acid esters, and ethylhexanoic acid esters. Examples of the alcohol component in the fatty acid ester include: monohydric alcohols such as methanol, ethanol, propanol, butanol, pentanol, hexanol, heptanol, octanol, decanol, 2-ethylhexanol, lauryl alcohol, myristyl alcohol, cetyl alcohol, stearyl alcohol, isostearyl alcohol, oleyl alcohol, behenyl alcohol, isopropyl alcohol, isobutyl alcohol, and isodecyl alcohol; dihydric alcohols such as ethylene glycol, propylene glycol, butylene glycol, diethylene glycol, and triethylene glycol; trihydric alcohols such as glycerin, trimethylolpropane, and triethanolamine; tetrahydric alcohols such as pentaerythritol, diglycerin, xylose, and sorbitan; pentahydric alcohols such as xylitol, triglycerin, glucose, and fructose; hexahydric alcohols such as sorbitol, malbitol, and tetraglycerin; polyglycerins having more than six hydroxyl groups; disaccharides such as sucrose, trehalose, and lactose; trisaccharides such as maltotriose, and the like.

Examples of the acrylic resin include polymers including a repeating unit of the following formula (5).

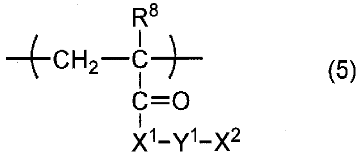

In the formula, R⁸ represents a hydrogen atom, a methyl group, or an ethyl group; X¹ represents an oxygen atom or an imino group; when X¹ is an oxygen atom, -Y¹-X² represents a hydrogen atom or an alkyl group, isobornyl group, -(CH₂)ₚ-N(R⁹)₂, -(CH₂)ₚ-N⁺(R⁹)₃, -(CH₂)ₚ-OR⁹, -C(CH₃)₂-N(R⁹)₂ or -C(CH₃)₂-OR⁹ (p represents a number from 1 or more to 3 or less, and R⁹ represents a hydrogen atom or an alkyl group having 1 or more and 4 or less carbon atoms) having 1 or more and 24 or less carbon atoms, or Y¹ represents a group consisting of an oxyethylene group and/or an oxypropylene group and X² represents a hydrogen atom, methyl group or ethyl group; and when X¹ represents NH, Y¹ represents a divalent saturated hydrocarbon group having 1 or more and 3 or less carbon atoms, and X² represents -N(R⁹)₂ (R⁹ is the same as described above).

Examples of such acrylic resins include poly(methacryloyloxyethyltrimethyl ammonium chloride) (INCI name: polyquaternium-37, for example, Cosmedia Ultragel 300 manufactured by BASF), N,N-dimethylaminoethyl methacrylate-vinylpyrrolidone copolymer diethyl sulfate salt (INCI name: polyquaternium-11, for example, Gafquat 734, Gafquat 755N, and Gafquat 755N-O manufactured by ISP), and acrylates / C₁₋₁₈ alkyl acrylates / C₁₋₈ alkyl acrylamide copolymer (for example, Plus Size L-9909B manufactured by GOO Chemical Co., Ltd.; 2-amino-2-methyl-1-propyl alcohol neutralizer of the polymer).

Among these components (B), preferable examples include polysilicone-9, polyquaternium-37 and polyethylene glycol.

The content of the component (B) in the fiber treatment agent composition of the present invention is, from the viewpoint of excellent expression of a color deepening effect, 0.05% by mass or more, preferably 0.5% by mass or more, more preferably 1.0% by mass or more, and further preferably 1.5% by mass or more, and from the viewpoint of excellent film formability and the accompanying expression of a deeper color of the fiber surface, 10.0% by mass or less, preferably 7.0% by mass or less, more preferably 5.0% by mass or less, and further preferably 2.5% by mass or less.

The mass ratio of the component (B) to the component (A), (B)/(A), in the fiber treatment agent composition of the present invention is, from the viewpoint of excellent film formability and accompanying expression of a color deepening effect, 150 or less, preferably 100 or less, and more preferably 10 or less, and from the viewpoint of excellent expression of a color deepening effect, preferably 0.05 or more, and more preferably 3 or more.

### [Component (C): Micelle formation inhibitor]

The epoxyaminosilane copolymer, such as polysilicone-29, among the component (A) has the characteristic of forming molecular aggregates (micelles) in water due to the fact that it has a hydrophobic polysiloxane moiety and a hydrophilic polyether moiety, and thus is capable of forming micelles. In addition, when used together with an agent (the component (C)) which inhibits that micelle formation ability, the epoxyaminosilane copolymer can improve the effect of imparting hydrophobicity to the fibers and reducing friction, and further improve the durability of those effects.

Examples of the micelle formation inhibitor of the component (C) include the following (C1) to (C3).
(C1) an organic compound having a hydrogen bond term of the Hansen solubility parameter of 10.0 MPa^{1/2} or more and 15.8 MPa^{1/2} or less (excluding organic compounds corresponding to (C3));
(C2) a compound selected from the group consisting of ethanol, triethylene glycol, pentylene glycol, methyl propanediol, diethanolamine, and N-methyldiethanolamine; and
(C3) an organic salt.

In the present invention, the hydrogen bond term of the Hansen solubility parameter refers to δ_{H} (MPa^{1/2}) (energy term for intermolecular hydrogen bonds) calculated by using a software package HSPiP 4th Edition 4.1.07 according to Hansen Solubility Parameters: A User's Handbook, CRC Press, Boca Raton Fla., 2007, in DIY program at 25°C.

Examples of the organic compound having a hydrogen bond term of the Hansen solubility parameter of 10.0 MPa^{1/2} or more and 15.8 MPa^{1/2} or less (C1) of the component (C) include organic compounds such as an aliphatic alcohol having a linear or branched alkyl group having 2 or more and 8 or less carbon atoms and one or more hydroxyl groups, an aromatic alcohol, an ether alcohol, an N-alkylpyrrolidone, an acyclic ester and an alkylamine optionally having a hydroxyl group.

Specific examples of the compounds having a hydrogen bond term of the Hansen solubility parameter of 10.0 MPa^{1/2} or more and 15.8 MPa^{1/2} or less will be described below. Numbers in parentheses for the respective examples represent the hydrogen bond term calculated by the above method.

Examples of the aliphatic alcohols having a linear or branched alkyl group having 2 or more and 8 or less carbon atoms and one or more hydroxyl groups: a lower alkanol such as 1-propanol (14.7), 2-propanol (14.3), 1-butanol (15.2) and 2-butanol (12.4); a polyhydric alcohol such as hexylene glycol (15.0), octanediol (14.5) and decanediol (12.8)

Examples of the aromatic alcohols: benzyl alcohol (12.4), cinnamyl alcohol (10.9), phenethyl alcohol (11.4), p-anisyl alcohol (12.1), p-methylbenzyl alcohol (11.2), phenoxyethanol (12.2), 2-benzyloxyethanol (10.8) and 2-phenyl-1-propanol (10.2)

Examples of the ether alcohols: ethylene glycol monoethyl ether (15.7), ethylene glycol monobutyl ether (13.0), diethylene glycol monomethyl ether (13.1), diethylene glycol monoethyl ether (11.9), diethylene glycol monobutyl ether (11.7) and ethylhexyl glyceryl ether (13.1)

Examples of the N-alkylpyrrolidones: N-(2-hydroxyethyl)-2-pyrrolidone (13.5)

Examples of the acyclic esters: ethyl lactate (14.4) and butyl lactate (11.9)

Examples of the alkylamines optionally having a hydroxyl group: methylamine (13.0), ethylamine (10.2), N,N-dimethylmonoethanolamine (13.1) and aminomethylpropanol (14.4)

Among them, from the viewpoint of exerting ability to inhibit formation of micelles, those having a hydrogen bond term of the Hansen solubility parameter of 15.5 MPa^{1/2} or less are preferred, those having that of 15.0 MPa^{1/2} or less are more preferred, those having that of 13.0 MPa^{1/2} or less are further preferred, and those having that of 12.5 MPa^{1/2} or less are further preferred. Those having a hydrogen bond term of the Hansen solubility parameter of 10.5 MPa^{1/2} or more are preferred, those having that of 11.0 MPa^{1/2} or more are more preferred, those having that of 11.5 MPa^{1/2} or more are further preferred, and those having that of 12.0 MPa^{1/2} or more are further preferred from the same point of view. In particular, an aromatic alcohol and an ether alcohol are preferred, and at least one selected from the group consisting of benzyl alcohol (12.4), phenethyl alcohol (11.4), phenoxyethanol (12.2) and 2-benzyloxyethanol (10.8) is more preferred.

Among the component (C), the compound selected from ethanol, triethylene glycol, pentylene glycol, methylpropanediol, diethanolamine and N-methyldiethanolamine (C2) has the ability to inhibit formation of micelles, although having a hydrogen bond term of the Hansen solubility parameter of more than 15.8 MPa^{1/2}.

At least one selected from the group consisting of a guanidine salt and an arginine salt, which have a guanidinium group, is preferred as the organic salt (C3) of the component (C). Examples of the guanidine salts include at least one selected from the group consisting of guanidine hydrochloride, guanidine nitrate, guanidine phosphate, guanidine thiocyanate, guanidine carbonate, guanidine acetate, guanidine sulfate, guanidine sulfamate, aminoguanidine hydrochloride, and aminoguanidine sulfate. Examples of the arginine salts include at least one selected from the group consisting of arginine hydrochloride, arginine nitrate, arginine phosphate, arginine thiocyanate, arginine carbonate, arginine acetate, arginine sulfate, arginine sulfamate, arginine glutamate, and arginine aspartate.

The component (C) may be used alone, or two or more of the component (C) may be used in combination. For example, benzyl alcohol and phenethyl alcohol may be selected from (C1) and used in combination. Further, benzyl alcohol may be selected from (C1) and ethanol may be selected from (C2) and these may be used in combination. The total amount of (C1) and (C2) in the component (C) is, from the viewpoint of improving the use impression, preferably 90% by mass or more, more preferably 95% by mass or more, further preferably 98% by mass or more, and even more preferably substantially 100% by mass.

More specifically, the component (C) is, from the viewpoint of suppression of formation of micelles in the epoxyaminosilane copolymer of the component (A) to facilitate adsorption to the fiber, impart sufficient hydrophobicity and friction-lowering effects in a short time and maintain these effects for a long time after the treatment, preferably at least one selected from the group consisting of ethanol, benzyl alcohol, triethylene glycol, pentylene glycol, methyl propanediol, phenoxyethanol, ethyl lactate, diethanolamine and a guanidine salt, more preferably at least one selected from the group consisting of ethanol, benzyl alcohol, phenoxyethanol, ethyl lactate, diethanolamine and a guanidine salt, and further preferably at least one selected from the group consisting of ethanol, benzyl alcohol and phenoxyethanol.

The content of the component (C) in the fiber treatment agent composition of the present invention is, from the viewpoint of maintaining the effect of imparting hydrophobicity to the fibers and the effect of lowering friction for a long time, preferably 5.0% by mass or more, preferably more 6.0% by mass or more, further preferably 7.0% by mass or more, further more preferably 10.0% by mass or more, even more preferably 12.0% by mass or more, and still yet even more preferably 15.0% by mass or more. In addition, from the viewpoint of improving storage stability, the content is preferably 96.8% by mass or less, more preferably 95.0% by mass or less, further preferably 90.0% by mass or less, further more preferably 80.0% by mass or less, even more preferably 70.0% by mass or less, and still yet even more preferably 60.0% by mass or less.

The mass ratio of the component (C) to the epoxyaminosilane copolymer, (C)/(epoxyaminosilane copolymer), is, from the viewpoint of producing a sufficient effect of inhibiting formation of micelles, preferably 1 or more, more preferably 5 or more, further preferably 6 or more, and further more preferably 7 or more. In addition, from the viewpoint of improving storage stability, the mass ratio is preferably 2,000 or less, more preferably 1,000 or less, further preferably 500 or less, and further more preferably 200 or less.

### [Water]

The fiber treatment agent composition of the present invention preferably contains water as a solvent. The content of the water in the fiber treatment agent composition of the present invention is, from the viewpoint of facilitating application onto the fibers, preferably 10% by mass or more, more preferably 15% by mass or more, further preferably 20% by mass or more. In addition, from the viewpoint of facilitating drying of the fibers after the application, the content is preferably 90% by mass or less, more preferably 85% by mass or less, and further preferably 80% by mass or less.

### [pH and pH Adjuster]

The pH of the fiber treatment agent composition of the present invention is preferably in the following range from the viewpoint of an increase in the reaction rate of the trialkoxysilane moiety of the component (A) in the acidic region or the basic region. When the pH of the fiber treatment agent composition of the present invention is in the acidic region, the pH is preferably 1 or more, more preferably 1.5 or more, and further preferably 2 or more, and preferably 5 or less, more preferably 4.0 or less, and further preferably 3.5 or less. Furthermore, when the pH of the fiber treatment agent composition of the present invention is in the basic region, the pH is preferably 7 or more, more preferably 7.5 or more, and further preferably 8.0 or more, and preferably 11 or less, more preferably 10.5 or less, and further preferably 10 or less. To adjust the pH of the fiber treatment agent composition of the present invention to the above range, the fiber treatment agent composition of the present invention may contain a pH adjuster as necessary. An alkali agent including alkanolamines such as monoethanolamine, isopropanolamine, 2-amino-2-methylpropanol and 2-aminobutanol, or a salt thereof; alkanediamines such as 1,3-propanediamine, or a salt thereof; carbonates such as guanidine carbonate, sodium carbonate, potassium carbonate, sodium hydrogen carbonate and potassium hydrogen carbonate; and hydroxides such as sodium hydroxide and potassium hydroxide may be used as the pH adjuster. Furthermore, acidic agents such as inorganic acids such as hydrochloric acid and phosphoric acid, hydrochlorides such as monoethanolamine hydrochloride; phosphates such as potassium dihydrogen phosphate and disodium hydrogen phosphate, and organic acids such as lactic acid, malic acid, and succinic acid may be used.

### [Optional components]

The fiber treatment agent composition of the present invention may also contain, in addition to the above components, a component usually blended in a fiber treatment agent composition as necessary. Examples thereof include an anti-dandruff agent; a vitamin preparation; a microbicide; an anti-inflammatory agent; an antiseptic; a chelating agent; a moisturizer; a colorant such as a dye or a pigment; extracts; a pearling agent; a perfume; an ultraviolet absorber; an antioxidant; a photocatalyst; shea butter; rose water; sunflower oil; orange oil; eucalyptus oil; and a surfactant. Examples of the photocatalysts include metal oxides such as titanium oxide and tungsten oxide, aromatic hydroxy compounds such as 8-hydroxyquinoline, 7-cyano-2-naphthol and 8-quinolinol-1-oxide, sulfonated pyrene compounds, onium salts, diazomethane derivatives, bissulfone derivatives, disulfono derivatives, nitrobenzyl sulfonate derivatives, sulfonic acid ester derivatives, and a sulfonic acid ester of N-hydroxyimide. Any one of a cationic surfactant, an anionic surfactant, an amphoteric surfactant and a nonionic surfactant may be used as the surfactant. Examples of the cationic surfactants include an alkylamine salt and an alkyl quaternary ammonium salt. Examples of the anionic surfactants include an alkyl sulfonate, an alkyl carboxylate, an alkyl ether sulfonate and an alkyl ether carboxylate. Examples of the amphoteric surfactants include imidazoline, carbobetaine, amidobetaine, sulfobetaine, hydroxysulfobetaine and amidosulfobetaine. Examples of the nonionic surfactants include esters such as a glycerol fatty acid ester, a sorbitan fatty acid ester and a sucrose fatty acid ester, and ethers such as a polyoxyethylene alkyl ether, a polyoxyethylene alkylphenyl ether, a polyoxyethylene polyoxypropylene alkyl ether and polyoxyethylene polyoxypropylene alkyl phenyl ether.

Specifically, polyols such as propylene glycol and glycerol may be used for the purpose of, for example, moisturization. The content of these components in the fiber treatment agent composition of the present invention is preferably 20% by mass or less, more preferably 10% by mass or less, and further preferably 5% by weight or less. The amount of the photocatalyst in the fiber treatment agent composition of the present invention is, from the viewpoint of maintaining storage stability of the cosmetic composition, preferably 2% by mass or less, more preferably 1% by mass or less, further preferably 0.1% by weight or less, and further more preferably substantially 0% by mass. The amount of the surfactant in the fiber treatment agent composition of the present invention is preferably 2% by mass or less, more preferably 1% by mass or less, further preferably 0.1% by weight or less, and further more preferably substantially 0% by mass from the viewpoint of maintaining persistent effects.

### [Agent form and the like]

The agent form of the fiber treatment agent composition of the present invention may be, for example, a liquid, an emulsion, a cream, a gel, a paste, a mousse, an aerosol. From the viewpoint of increasing the drying rate to promote formation of the film, the agent form is preferably a liquid, a gel, a paste, a mousse and an aerosol. If the agent form is an aerosol, the content of each component described above and the pH of the fiber treatment agent composition mean the content of each component in a stock solution not containing a propellant and the pH of the stock solution, respectively.

### [Fibers to be treated]

Examples of the fibers to be treated by the fiber treatment agent composition of the present invention include keratin fibers such as hair and fiber products such as cloth and clothes, but hair is preferred.

### [Method of use]

The fiber treatment agent composition of the present invention may be used by a method in which the composition is rinsed off after being applied to the fiber, or a method in which the composition is applied to the fiber and dried without being rinsed off. It is preferable that the composition be used by the method in which the composition is applied to the fiber and then dried without being rinsed off in order to increase the effect of the present invention.

The amount of the fiber treatment agent composition of the present invention to be applied to the fiber is determined relative to the mass of the fiber so that the bath ratio (the mass of the fiber treatment agent composition/the mass of the fiber) is preferably 0.001 or more, more preferably 0.005 or more, and further preferably 0.01 or more, and preferably 100 or less, more preferably 10 or less, and further preferably 1 or less.

In the present invention, the fiber treatment can be performed by, after applying the composition containing the component (A) to the fiber surface, without drying, applying the composition containing the component (B) to the applied portion, and then drying without rinsing off. Further, the fiber treatment can also be performed by, after applying the composition containing the component (B) to the fiber surface, without drying, applying the composition containing the component (A) to the applied portion, and then drying without rinsing off.

In these cases, the content of the component (A) in the composition containing the component (A) and the content of the component (B) in the composition containing the component (B) can be the same as the content of the component (A) or the component (B) in the fiber treatment agent composition of the present invention described above. These compositions can further contain the component (C) and other components with water as a solvent. Further, the amount of these compositions applied to the fiber can be the same as the bath ratio described above.

In relation to the embodiment described above, preferred aspects of the present invention are now further disclosed.
<1> A fiber treatment agent composition comprising the following components (A) and (B), wherein a content of the component (A) is 0.05% by mass or more and 5.0% by mass or less, a content of the component (B) is 0.05% by mass or more and 10.0% by mass or less, and a mass ratio of the component (B) to the component (A), (B)/(A), is 150 or less,
   (A) a self-crosslinking compound; and
   (B) one or more color deepening agents selected from the group consisting of a nonvolatile silicone, a polyhydric alcohol polymer, a fatty acid ester, and an acrylic resin (excluding agents corresponding to the component (A)).
<2> The fiber treatment agent composition according to <1>, wherein the component (A) is preferably one or more selected from the group consisting of the following components (A1) to (A3):
   (A1) an alkoxysilyl group-containing silicone;
   (A2) an alkoxysilyl group-containing acrylate compound; and
   (A3) an alkoxysilyl group-containing alkylamine.
<3> The fiber treatment agent composition according to <2>, wherein the component (A1) is preferably a silicone in which an alkoxysilyl group -SiR¹ₙ(OR²)₃₋ₙ (wherein R¹ and R² independently represent a monovalent hydrocarbon group, and n represents a number from 0 to 2) is bonded to an organopolysiloxane residue.
<4> The fiber treatment agent composition according to <3>, wherein the component (A1) is preferably an epoxyaminosilane copolymer, which is a reaction product of the following compounds (a) to (d):
   (a) a polysiloxane having at least two oxiranyl groups or oxetanyl groups;
   (b) a polyether having at least two oxiranyl groups or oxetanyl groups;
   (c) aminopropyltrialkoxysilane; and
   (d) a compound selected from the group consisting of the following primary and secondary amines:
      primary amines: methylamine, ethylamine, propyleneamine, ethanolamine, isopropylamine, butylamine, isobutylamine, hexylamine, dodecylamine, oleylamine, aniline, aminopropyltrimethylsilane, aminopropyltriethylsilane, aminomorpholine, aminopropyldiethylamine, benzylamine, naphthylamine, 3-amino-9-ethylcarbazole, 1-aminoheptafluorohexane, and 2,2,3,3,4,4,5,5,6,6,7,7,8,8,8-pentadecafluoro-1-octaneamine; and
      secondary amines: methylethylamine, methyloctadecylamine, diethanolamine, dibenzylamine, dihexylamine, dicyclohexylamine, piperidine, pyrrolidinephthalimide, and polymer amines.
<5> The fiber treatment agent composition according to <4>, wherein the compound (a) is preferably a compound of the following formula (1): 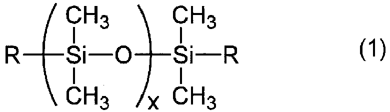 wherein R represents a hydrocarbon group which has an oxiranyl group or oxetanyl group at an end, optionally contains a hetero atom, and has 1 to 6 carbon atoms, and x represents a number from 1 to 1000.
<6> The fiber treatment agent composition according to <4> or <5>, wherein the compound (b) is preferably a compound of the following formula (2): 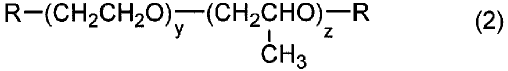 wherein R has the same meaning as described above, y represents a number from 1 to 100, z represents a number from 0 to 100, and y + z is a number from 1 to 200.
<7> The fiber treatment agent composition according to any one of <4> to <6>, wherein the compound (c) is preferably at least one selected from the group consisting of aminopropyltrimethoxysilane, aminopropyltriethoxysilane, aminopropyltripropoxysilane, aminopropyltriisopropoxysilane, aminopropyltributoxysilane, and aminopropyltri-tert-butoxysilane.
<8> The fiber treatment agent composition according to any one of <4> to <7>, wherein the compound (d) is preferably a primary amine, and more preferably is at least selected from the group consisting of aminopropyldiethylamine, aminopropyldimethylamine, and aminopropyldibutylamine.
<9> The fiber treatment agent composition according to any one of <4> to <8>, wherein the component (A1) is preferably polysilicone-29.
<10> The fiber treatment agent composition according to <3>, wherein the component (A1) is preferably an (amodimethicone/morpholinomethyl silsesquioxane) copolymer.
<11> The fiber treatment agent composition according to <2>, wherein the component (A2) is preferably a compound in which an alkoxysilyl group -SiR¹ₙ(OR²)₃₋ₙ (wherein R¹ and R² independently represent a monovalent hydrocarbon group, and n represents a number from 0 to 2) is bonded to a group of the following formula (3): 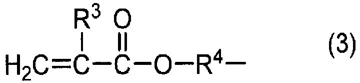 wherein R³ represents a hydrogen atom or a methyl group, and R⁴ represents a divalent saturated hydrocarbon group having 1 to 6 carbon atoms.
<12> The fiber treatment agent composition according to <11>, wherein the component (A2) is preferably a silane coupling agent selected from the group consisting of 3-methacryloxypropyldimethoxysilane, 3-methacryloxypropyltrimethoxysilane, 3-methacryloxypropylmethyldiethoxysilane, 3-methacryloxypropyltriethoxysilane, and 3-acryloxypropyltrimethoxysilane.
<13> The fiber treatment agent composition according to <2>, wherein the component (A3) is preferably a compound in which an alkoxysilyl group -SiR¹ₙ(OR²)₃₋ₙ (wherein R¹ and R² independently represent a monovalent hydrocarbon group, and n represents a number from 0 to 2) is bonded to a group of the following formula (4): 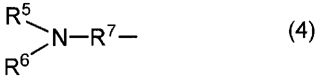 wherein R⁵ and R⁶ represent a hydrogen atom or a hydrocarbon group optionally substituted with an amino group, or R⁵ and R⁶ jointly form an alkylidene group, and R⁷ represents a divalent hydrocarbon group having 1 to 6 carbon atoms.
<14> The fiber treatment agent composition according to <13>, wherein the component (A3) is preferably selected from the group consisting of a hydrochloride of N-2-(aminoethyl)-3-aminopropylmethyldimethoxysilane, N-2-(aminoethyl)-3-aminopropyltrimethoxysilane, 3-aminopropyltrimethoxysilane, 3-aminopropyltriethoxysilane, 3-triethoxysilyl-N-(1,3-dimethylbutylidene)propylamine, N-phenyl-3-aminopropyltrimethoxysilane, and N-(vinylbenzyl)-2-aminoethyl-3-aminopropyltrimethoxysilane, and more preferably at least one selected from the group consisting of 3-aminopropyl-triethoxysilane, 3-aminopropylmethyldiethoxysilane, N-(2-aminoethyl)-3-aminopropyltriethoxysilane, and 3-(2-aminoethylamino)propylmethyldiethoxysilane.
<15> The fiber treatment agent composition according to any one of <1> to <14>, wherein the content of the component (A) is preferably 0.1% by mass or more, more preferably 0.2% by mass or more, and further preferably 0.25% by mass or more, and preferably 3.0% by mass or less, more preferably 1.0% by mass% or less, and further preferably 0.5% by mass or less.
<16> The fiber treatment agent composition according to any one of <1> to <15>, wherein the component (B) is preferably at least one nonvolatile silicone selected from the group consisting of dimethylpolysiloxane, amino-modified polysiloxane, phenyl-modified polysiloxane, polyglycerin-modified polysiloxane, silicone resin, acrylic-modified polysiloxane, alkyl-modified polysiloxane, methacryl-modified polysiloxane, carboxyl-modified polysiloxane, aminopolyether-modified polysiloxane, oxazoline-modified polysiloxane, polyether-modified polysiloxane, fluorine-modified polysiloxane, mercapto-modified polysiloxane, hydrogen-modified polysiloxane, aralkyl-modified polysiloxane, epoxy-modified polysiloxane, and hydroxy-modified polysiloxane.
<17> The fiber treatment agent composition according to any one of <1> to <15>, wherein the component (B) is preferably at least one polyhydric alcohol polymer selected from the group consisting of diethylene glycol, dipropylene glycol, triethylene glycol, polypropylene glycol, tetraethylene glycol, diglycerin, polyethylene glycol, triglycerin, tetraglycerin, and polyglycerin.
<18> The fiber treatment agent composition according to any one of <1> to <15>, wherein the component (B) is preferably at least one fatty acid ester selected from the group consisting of lauric acid esters, myristic acid esters, palmitic acid esters, oleic acid esters, phthalic acid esters, adipic acid esters, caprylic acid esters, capric acid esters, behenic acid esters, methacrylic acid esters, and ethylhexanoic acid esters.
<19> The fiber treatment agent composition according to <18>, wherein the alcohol component in the fatty acid ester is preferably at least one selected from the group consisting of methanol, ethanol, propanol, butanol, pentanol, hexanol, heptanol, octanol, decanol, 2-ethylhexanol, lauryl alcohol, myristyl alcohol, cetyl alcohol, stearyl alcohol, isostearyl alcohol, oleyl alcohol, behenyl alcohol, isopropyl alcohol, isobutyl alcohol, isodecyl alcohol, ethylene glycol, propylene glycol, butylene glycol, diethylene glycol, triethylene glycol, glycerin, trimethylolpropane, triethanolamine, pentaerythritol, diglycerin, xylose, sorbitan, xylitol, triglycerin, glucose, fructose, sorbitol, malbitol, tetraglycerin, polyglycerins having more than six hydroxyl groups, sucrose, trehalose, lactose, and maltotriose.
<20> The fiber treatment agent composition according to any one of <1> to <15>, wherein the component (B) is preferably a polymer comprising a repeating unit of the following formula (5): 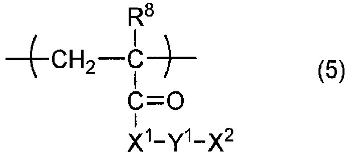 wherein R⁸ represents a hydrogen atom, a methyl group, or an ethyl group, X¹ represents an oxygen atom or an imino group, when X¹ is an oxygen atom, -Y¹-X² represents a hydrogen atom or an alkyl group, isobornyl group, -(CH₂)ₚ-N(R⁹)₂, -(CH₂)ₚ-N⁺(R⁹)₃, -(CH₂)ₚ-OR⁹, - C(CH₃)₂-N(R⁹)₂ or -C(CH₃)₂-OR⁹ (p represents a number from 1 or more to 3 or less, and R⁹ represents a hydrogen atom or an alkyl group having 1 or more and 4 or less carbon atoms) having 1 or more and 24 or less carbon atoms, or when Y¹ represents a group consisting of an oxyethylene group and/or an oxypropylene group, X² represents a hydrogen atom, methyl group or ethyl group and X¹ represents NH, Y¹ represents a divalent saturated hydrocarbon group having 1 or more and 3 or less carbon atoms, and X² represents -N(R⁹)₂ (R⁹ is the same as described above).
<21> The fiber treatment agent composition according to <20>, wherein the component (B) is preferably at least one selected from the group consisting of poly(methacryloyloxyethyltrimethyl ammonium chloride) (INCI name: polyquaternium-37), N,N-dimethylaminoethyl methacrylate-vinylpyrrolidone copolymer diethyl sulfate salt (INCI name: polyquaternium-11), and acrylates / C₁₋₁₈ alkyl acrylates / C₁₋₈ alkyl acrylamide copolymer.
<22> The fiber treatment agent composition according to any one of <1> to <21>, wherein the component (B) is preferably at least one selected from the group consisting of polysilicone-9, polyquaternium-37 and polyethylene glycol.
<23> The fiber treatment agent composition according to any one of <1> to <22>, wherein the content of the component (B) is preferably 0.5% by mass or more, more preferably 1.0% by mass or more, and further preferably 1.5 by mass or more, and preferably 7.0% by mass or less, more preferably 5.0 by mass% or less, and further preferably 2.5% by mass or less.
<24> The fiber treatment agent composition according to any one of <1> to <23>, wherein the mass ratio of the component (B) to the component (A), (B)/(A), is 150 or less, preferably 100 or less, and more preferably 10 or less, and preferably 0.05 or more, and more preferably 3 or more.
<25> The fiber treatment agent composition according to any one of <4> to <9>, preferably further containing at least one micelle formation inhibitor selected from the group consisting of the following (C1) to (C3):
   (C1) an organic compound having a hydrogen bond term of the Hansen solubility parameter of 10.0 MPa^{1/2} or more and 15.8 MPa^{1/2} or less (excluding organic compounds corresponding to (C3));
   (C2) a compound selected from the group consisting of ethanol, triethylene glycol, pentylene glycol, methyl propanediol, diethanolamine, and N-methyldiethanolamine; and
   (C3) an organic salt.
<26> The fiber treatment agent composition according to <25>, wherein the component (C1) is preferably one or two or more selected from the group consisting of 1-propanol, 2-propanol, 1-butanol, 2-butanol, hexylene glycol, octanediol, decanediol, benzyl alcohol, cinnamyl alcohol, phenethyl alcohol, p-anisyl alcohol, p-methylbenzyl alcohol, phenoxyethanol, 2-benzyloxyethanol, 2-phenyl-1-propanol, ethylene glycol monoethyl ether, ethylene glycol monobutyl ether, diethylene glycol monomethyl ether, diethylene glycol monoethyl ether, diethylene glycol monobutyl ether, ethylhexyl glyceryl ether, N-(2-hydroxyethyl)-2-pyrrolidone, ethyl lactate, butyl lactate, methylamine, ethylamine, N,N-dimethylmonoethanolamine and aminomethylpropanol, and more preferably at least one selected from the group consisting of benzyl alcohol, phenethyl alcohol, phenoxyethanol, and 2-benzyloxyethanol.
<27> The fiber treatment agent composition according to <25>, wherein the component (C3) is preferably at least one selected from the group consisting of a guanidine salt and an arginine salt, which have a guanidinium group, and more preferably guanidine hydrochloride, guanidine nitrate, guanidine phosphate, guanidine thiocyanate, guanidine carbonate, guanidine acetate, guanidine sulfate, guanidine sulfamate, aminoguanidine hydrochloride, aminoguanidine sulfate, arginine hydrochloride, arginine nitrate, arginine phosphate, arginine thiocyanate, arginine carbonate, arginine acetate, arginine sulfate, arginine sulfamate, arginine glutamate, and arginine aspartate.
<28> The fiber treatment agent composition according to any one of <25> to <27>, wherein a total amount of (C1) and (C2) in the component (C) is preferably 90% by mass or more, more preferably 95% by mass or more, further preferably 98% by mass or more, and further more preferably substantially 100% by mass.
<29> The fiber treatment agent composition according to any one of <25> to <28>, wherein the content of the component (C) is preferably 5.0% by mass or more, preferably more 6.0% by mass or more, further preferably 7.0% by mass or more, further more preferably 10.0% by mass or more, even more preferably 12.0% by mass or more, and still yet even more preferably 15.0% by mass or more, and preferably 96.8% by mass or less, more preferably 95.0% by mass or less, further preferably 90.0% by mass or less, further more preferably 80.0% by mass or less, even more preferably 70.0% by mass or less, and still yet even more preferably 60.0% by mass or less.
<30> The fiber treatment agent composition according to any one of <25> to <29>, wherein a mass ratio of the component (C) to the epoxyaminosilane copolymer, (C)/(epoxyaminosilane copolymer), is preferably 1 or more, more preferably 5 or more, further preferably 6 or more, and further more preferably 7 or more, and preferably 2,000 or less, more preferably 1,000 or less, further preferably 500 or less, and further more preferably 200 or less.
<31> The fiber treatment agent composition according to any one of <1> to <30>, wherein pH is preferably 1 or more, more preferably 1.5 or more, and further preferably 2 or more, and preferably 5 or less, more preferably 4.0 or less, and further preferably 3.5 or less.
<32> The fiber treatment agent composition according to any one of <1> to <30>, wherein pH is preferably 7 or more, more preferably 7.5 or more, and further preferably 8.0 or more, and preferably 11 or less, more preferably 10.5 or less, and further preferably 10 or less.
<33> A fiber treatment method comprising applying the fiber treatment agent composition according to any one of <1> to <32> to a fiber surface, and then drying without rinsing off.
<34> The fiber treatment method according to <33>, wherein an amount of the fiber treatment agent composition to be applied to the fiber is, based on a bath ratio relative to the mass of the fiber [(mass of the fiber treatment agent composition)/(mass of the fiber)] is preferably 0.001 or more, more preferably 0.005 or more, and further preferably 0.01 or more, and preferably 100 or less, more preferably 10 or less, and further preferably 1 or less.
<35> A fiber treatment method comprising applying a composition containing the following component (A) to a fiber surface, then, without drying, applying a composition containing the following component (B) to the applied portion, and then drying without rinsing off:
   (A) a self-crosslinking compound; and
   (B) one or more color deepening agents selected from the group consisting of a nonvolatile silicone, a polyhydric alcohol polymer, a fatty acid ester, and an acrylic resin (excluding agents corresponding to the component (A)).
<36> A fiber treatment method comprising applying a composition containing the following component (B) to a fiber surface, then, without drying, applying a composition containing the following component (A) to the applied portion, and then drying without rinsing off:
   (A) a self-crosslinking compound; and
   (B) one or more color deepening agents selected from the group consisting of a nonvolatile silicone, a polyhydric alcohol polymer, a fatty acid ester, and an acrylic resin (excluding agents corresponding to the component (A)).
<37> A fiber treatment agent composition comprising the following components (A) and (B), wherein a content of the component (A) is 0.25% by mass or more and 0.5% by mass or less, and a content of the component (B) is 1.5% by mass or more and 2.5% by mass or less:
   (A) polysilicone-29, and
   (B) polysilicone-9.
<38> A fiber treatment agent composition comprising the following components (A) and (B), wherein a content of the component (A) is 0.25% by mass or more and 0.5% by mass or less, and a content of the component (B) is 1.5% by mass or more and 2.5% by mass or less:
   (A) polysilicone-29, and
   (B) polyquaternium-37.
<39> A fiber treatment agent composition comprising the following components (A) and (B), wherein a content of the component (A) is 0.25% by mass or more and 0.5% by mass or less, and a content of the component (B) is 1.5% by mass or more and 2.5% by mass or less:
   (A) polysilicone-29, and
   (B) polyethylene glycol.

### Examples

### Examples 1 to 16 and Comparative Examples 1 to 7

Aqueous solutions having the composition shown in Tables 1 to 3 were prepared, and the film formability of these aqueous solutions, the color deepening effect when treated on hair, and the durability of the film were evaluated.

### (Method for preparing the aqueous solutions)

The predetermined amounts shown in Table 1 of distilled water and the respective components were weighed into a 200 mL beaker in which a stirring bar was placed, then mixed by stirred using a stirrer for 30 minutes or more until the solutions became homogeneous.

### (Method for evaluating film formability)

300 µL of each of the aqueous solutions was added dropwise onto a slide glass, and the slide glass was dried in an oven set at 90°C for 30 minutes. Then, after leaving at room temperature for about 10 minutes, the presence or absence of film formation was confirmed based on appearance and sensory evaluation. The evaluation was made on a scale of 1 to 5: dry films that were smooth, non-sticky, and did not adhere to the hands were evaluated as "5: very good", dry films that were non-sticky and did not adhere to the hands were evaluated as "4: good", dry films that were somewhat sticky were evaluated as "3: normal", films that had a portion that was not dry were evaluated as "2: bad", and films that had a large portion that was not dry were evaluated as "1: very bad".

### (Method for evaluating color deepening effect)

Damaged hair, that is, 3.0 g tresses of healthy hair all from Caucasian people that had then been treated once with powder bleach (GOLDWELL TOPCHIC), was used as the hair for evaluation.

Each of the aqueous solutions shown in Tables 1 to 3 was uniformly applied at a bath ratio of 0.2 with respect to the hair onto the tresses, which had been wetted with water (separate tresses were used for each aqueous solution), and the tresses were then dried completely using a dryer without rinsing off. A process starting from washing the tresses once using the plain shampoo shown below until completely drying using a dryer was repeated a total of three times.

| - Composition of plain shampoo (pH 6.9) | (% by mass) |
|---|---|
| Sodium polyoxyethylene lauryl ether sulfate (EMAL 170J, manufactured by Kao Corporation, active ingredient 70% by mass) | 13.0 |
| Coconut oil fatty acid monoethanolamide (AMISOL CME, manufactured by Kawaken Fine Chemicals Co., Ltd.) | 0.6 |
| Coconut oil fatty acid amidopropylcarbobetaine (AMPHITOL 55AB, manufactured by Kao Corporation, active ingredient 30% by mass) | 1.41 |
| Citric acid | Amount to adjust pH |
| Sodium benzoate | 0.3 |
| Purified water | Balance |

The Lab value, which is a color index, of each tress after a powder bleaching treatment was measured by using a color difference meter before and after treating the aqueous solution. A CR-400 from Konica Minolta was used for the color difference meter. The tresses were stroked a total of five times at a speed of one stroke per three seconds with a straightener set at 180°C, and the measurement was carried out with the surface in a straightened state. By using the measured L value, a five-stage evaluation was performed. A larger negative difference (ΔL) of the L value after the aqueous solution treatment with respect to before the treatment indicates a higher effect of deepening in color. The evaluation was made on a scale of 1 to 5: cases where ΔL was 2.5 or more were evaluated as "5: very high", cases where ΔL was less than 2.5 and 1.5 or more were evaluated as "4: high", cases where ΔL was less than 1.5 and 1.0 or more were evaluated as "3: normal", cases where ΔL was less than 1.0 and 0.5 or more were evaluated as "2: low", and cases where ΔL was less than 0.5 were evaluated as "1: very low".

### (Method for evaluating film durability)

1.0 g tresses of healthy hair all from Japanese people were used as the hair for evaluation.

Each of the aqueous solutions shown in Table 1 or 2 was applied at a bath ratio of 0.3 with respect to the hair onto the tresses, which had been wetted with water and then towel dried, and the tresses were then dried completely by using a dryer without rinsing off. The tresses were then washed once by using the above-described plain shampoo and completely dried by using a dryer.

A sensory evaluation of the tresses upon drying before and after the shampoo washing was carried out, and the difference in feel was compared.

Cases where the feel did not change even after the shampoo wash and where there was no hair catching from the middle to the hair ends were evaluated as "A", cases where there was slight catching were evaluated as "B", and cases where there was catching were evaluated as "C".

**[Table 1]**

| Content (% by mass; active amount for all) | | Example | | | | | | | | | | |
|---|---|---|---|---|---|---|---|---|---|---|---|---|
| | | 1 | 2 | 3 | 4 | 5 | 6 | 7 | 8 | 9 | 10 | 11 |
| (A) | Epoxyaminosilane copolymer (*1) | 0.5 | 0.05 | 5 | - | 0.5 | 0.5 | 0.5 | 0.5 | 0.5 | 0.1 | 5 |
| | 3-Aminopropyltriethoxysilane (*2) | - | - | - | 0.5 | - | - | - | - | - | - | - |
| (B) | Polysilicone-9 (*3) | 2 | 2 | 2 | 2 | 0.05 | 10 | 2 | 2 | 2 | 10 | 0.05 |
| | pH Adjusters (lactic acid, sodium hydroxide) | *4 | *4 | *4 | *4 | *4 | *4 | *4 | *4 | *4 | *4 | *4 |
| (C) | Ethanol | 30 | 30 | 30 | - | 30 | 30 | - | - | - | 30 | 30 |
| | Dipropylene glycol | - | - | - | - | - | - | - | 30 | - | - | - |
| | Hexylene glycol | - | - | - | - | - | - | - | - | 30 | - | - |
| | Water | balance | balance | balance | balance | balance | balance | balance | balance | balance | balance | balance |
| Mass ratio (B)/(A) | | 4 | 40 | 0.4 | 4 | 0.1 | 20 | 4 | 4 | 4 | 100 | 0.01 |
| pH | | 3.5 | 3.5 | 3.5 | 3.5 | 3.5 | 3.5 | 3.5 | 3.5 | 3.5 | 3.5 | 3.5 |
| Evaluations | Film formability | 5 | 4 | 4 | 5 | 5 | 4 | 5 | 5 | 5 | 4 | 4 |
| | Color deepening | 5 | 5 | 5 | 4 | 4 | 5 | 4 | 4 | 4 | 5 | 4 |
| | Film durability | A | A | A | B | A | A | B | A | A | A | A |

| | | | | | | | | | | | | |
|---|---|---|---|---|---|---|---|---|---|---|---|---|
| *1: Silsoft CLX-E (manufactured by Momentive Performance Materials, polysilicone-29: 15% by mass) *2: KBE-903 (manufactured by Shin-Etsu Chemical Co., Ltd.) *3: N-propionylethyleneimine/dimethylsiloxane copolymer described in Synthesis Example 2 of JP-A-2017-186286 *4: pH adjustment amount | | | | | | | | | | | | |

**[Table 2]**

| Content (% by mass; active amount for all) | | Example | | | | |
|---|---|---|---|---|---|---|
| | | 12 | 13 | 14 | 15 | 16 |
| (A) | Epoxyaminosilane copolymer (*1) | 0.5 | 0.5 | 0.5 | 0.5 | 0.5 |
| (B) | Copolymer of ethyleneoxide and propyleneoxide (*5) | 2 | - | - | - | - |
| | Copolymer of ethyleneoxide and propyleneoxide (*6) | - | 2 | - | - | - |
| | Poly(methacryloyloxyethyltrimethyl ammonium chloride) (*7) | - | - | 2 | - | - |
| | Polyoxyethylene sorbitan monooleate (*8) | - | - | - | 2 | - |
| | Polyoxyethylene sorbitan monolaurate (*9) | - | - | - | - | 2 |
| | pH Adjusters (lactic acid, sodium hydroxide) | *4 | *4 | *4 | *4 | *4 |
| (C) | Ethanol | 30 | 30 | 30 | 30 | 30 |
| | Water | balance | balance | balance | balance | balance |
| Mass ratio (B)/(A) | | 4 | 4 | 4 | 4 | 4 |
| pH | | 3.5 | 3.5 | 3.5 | 3.5 | 3.5 |
| Evaluations | Film formability(90 °C) | 5 | 4 | 5 | 5 | 4 |
| | Color deepening | 4 | 4 | 4 | 3 | 3 |
| | Film durability | B | B | A | B | B |

| | | | | | | |
|---|---|---|---|---|---|---|
| *1: Silsoft CLX-E (manufactured by Momentive Performance Materials, polysilicone-29: 15% by mass) *4: pH adjustment amount *5: Alkox EP1010N (manufactured by Meisei Chemical Works, Ltd., weight average molecular weight 100,000, EO/PO mass ratio = 90/10, random polymerization) *6: Alkox R-1000G (manufactured by Meisei Chemical Works, Ltd., weight average molecular weight from 250,000 to 400,000, EO/PO mass ratio = 90/10, random polymerization) *7: Cosmedia Ultragel 300 (manufactured by BASF; polyquaternium-37) *8: Rheodol TW-O120V (manufactured by Kao Corporation) *9: Emanon 1112 (manufactured by Kao Corporation) | | | | | | |

**[Table 3]**

| Content (% by mass; active amount for all) | | Comparative Example | | | | | | |
|---|---|---|---|---|---|---|---|---|
| | | 1 | 2 | 3 | 4 | 5 | 6 | 7 |
| (A) | Epoxyaminosilane copolymer (*1) | 0.5 | 0.01 | 10 | 0.5 | 0.5 | 5 | 0.05 |
| (B) | Polysilicone-9 (*3) | - | 2 | 2 | 0.01 | 20 | 0.025 | 10 |
| | pH Adjusters (lactic acid, sodium hydroxide) | *4 | *4 | *4 | *4 | *4 | *4 | *4 |
| (C) | Ethanol | 30 | 30 | 30 | 30 | 30 | 30 | 30 |
| | Water | balance | balance | balance | balance | balance | balance | balance |
| Mass ratio (B)/(A) | | 0 | 200 | 0.2 | 0.02 | 40 | 0.005 | 200 |
| pH | | 3.5 | 3.5 | 3.5 | 3.5 | 3.5 | 3.5 | 3.5 |
| Evaluations | Film formability | 5 | 1 | 2 | 5 | 2 | 4 | 1 |
| | Color deepening | 2 | 1 | 3 | 2 | 2 | 2 | 2 |
| | Film durability | A | B | B | A | B | A | B |

| | | | | | | | | |
|---|---|---|---|---|---|---|---|---|
| *1: Silsoft CLX-E (manufactured by Momentive Performance Materials, polysilicone-29: 15% by mass) *3: N-propionylethyleneimine/dimethylsiloxane copolymer described in Synthesis Example 2 of JP-A-2017-186286 *4: pH adjustment amount | | | | | | | | |

### Formulation Example 1 (Gel for leave-on hair fiber treatment; pH 3.5)

| | |
|---|---|
| | (% by mass; active amount for all) |
| Epoxyaminosilane copolymer | |
| (Silsoft CLX-E manufactured by Momentive Performance Materials was used, active amount of 15% by mass) | 3.0 |
| Ethanol | 30.0 |
| Poly(methacryloyloxyethyltrimethyl ammonium chloride) (Cosmedia Ultragel 300, manufactured by BASF) | 1.00 |
| Lactic acid | Amount to adjust pH |
| Water | Balance (*10) |

| | |
|---|---|
| *10 Including water and dipropylene glycol derived from Silsoft CLX-E | |

### Formulation Example 2 (Lotion for leave-on hair fiber treatment; pH 3.5)

| | |
|---|---|
| | (% by mass; active amount for all) |
| 3-acryloxypropyltrimethoxysilane | |
| (KBM-5103, manufactured by Shin-Etsu Chemical Co., Ltd.) | 3.0 |
| Ethanol | 30.0 |
| Poly(methacryloyloxyethyltrimethyl ammonium chloride) (Cosmedia Ultragel 300, manufactured by BASF) | 0.50 |
| Lactic acid | Amount to adjust pH |
| Water | Balance |

## Claims

1. A fiber treatment agent composition comprising the following components (A) and (B), wherein a content of the component (A) is 0.05% by mass or more and 5.0% by mass or less, a content of the component (B) is 0.05% by mass or more and 10.0% by mass or less, and a mass ratio of the component (B) to the component (A), (B)/(A), is 150 or less:
(A) a self-crosslinking compound; and
(B) one or more color deepening agents selected from the group consisting of a nonvolatile silicone, a polyhydric alcohol polymer, a fatty acid ester, and an acrylic resin (excluding agents corresponding to the component (A)).

2. The fiber treatment agent composition according to claim 1, wherein the component (A) is one or more selected from the group consisting of the following components (A1) to (A3):
(A1) an alkoxysilyl group-containing silicone;
(A2) an alkoxysilyl group-containing acrylate compound; and
(A3) an alkoxysilyl group-containing alkylamine.

3. The fiber treatment agent composition according to claim 1 or 2, wherein the mass ratio of the component (B) to the component (A), (B)/(A), is 0.05 or more and 150 or less.

4. The fiber treatment agent composition according to any one of claims 1 to 3, wherein a pH is 1 or more and 5 or less, or 7 or more and 11 or less.

5. The fiber treatment agent composition according to claim 2 or 3, wherein the component (A) is an alkoxysilyl group-containing silicone (A1).

6. The fiber treatment agent composition according to claim 5, wherein the component (A1) is an epoxyaminosilane copolymer, which is a reaction product of the following compounds (a) to (d):
(a) a polysiloxane having at least two oxiranyl groups or oxetanyl groups;
(b) a polyether having at least two oxiranyl groups or oxetanyl groups;
(c) aminopropyltrialkoxysilane; and
(d) a compound selected from the group consisting of the following primary and secondary amines:
primary amines: methylamine, ethylamine, propyleneamine, ethanolamine, isopropylamine, butylamine, isobutylamine, hexylamine, dodecylamine, oleylamine, aniline, aminopropyltrimethylsilane, aminopropyltriethylsilane, aminomorpholine, aminopropyldiethylamine, benzylamine, naphthylamine, 3-amino-9-ethylcarbazole, 1-aminoheptafluorohexane, and 2,2,3,3,4,4,5,5,6,6,7,7,8,8,8-pentadecafluoro-1-octaneamine; and
secondary amines: methylethylamine, methyloctadecylamine, diethanolamine, dibenzylamine, dihexylamine, dicyclohexylamine, piperidine, pyrrolidinephthalimide, and polymer amines.

7. The fiber treatment agent composition according to claim 6, wherein the component (A1) is polysilicone-29.

8. The fiber treatment agent composition according to claim 6 or 7, further comprising at least one micelle formation inhibitor selected from the group consisting of the following (C1) to (C3):
(C1) an organic compound having a hydrogen bond term of the Hansen solubility parameter of 10.0 MPa^{1/2} or more and 15.8 MPa^{1/2} or less (excluding organic compounds corresponding to (C3));
(C2) a compound selected from the group consisting of ethanol, triethylene glycol, pentylene glycol, methyl propanediol, diethanolamine, and N-methyldiethanolamine; and
(C3) an organic salt.

9. The fiber treatment agent composition according to claim 8, wherein a content of the micelle formation inhibitor is 5.0% by mass or more and 96.8% by mass or less.

10. The fiber treatment agent composition according to any one of claims 1 to 9, wherein the component (B) is one or more selected from the group consisting of polysilicone-9, polyquaternium-37, and polyethylene glycol.

11. A fiber treatment method comprising applying the fiber treatment agent composition according to any one of claims 1 to 10 to a fiber surface, and then drying without rinsing off.

12. A fiber treatment method comprising applying a composition containing the following component (A) to a fiber surface, then, without drying, applying a composition containing the following component (B) to the applied portion, and then drying without rinsing off:
(A) a self-crosslinking compound; and
(B) one or more color deepening agents selected from the group consisting of a nonvolatile silicone, a polyhydric alcohol polymer, a fatty acid ester, and an acrylic resin (excluding agents corresponding to the component (A)).

13. A fiber treatment method comprising applying a composition containing the following component (B) to a fiber surface, then, without drying, applying a composition containing the following component (A) to the applied portion, and then drying without rinsing off:
(A) a self-crosslinking compound; and
(B) one or more color deepening agents selected from the group consisting of a nonvolatile silicone, a polyhydric alcohol polymer, a fatty acid ester, and an acrylic resin (excluding agents corresponding to the component (A)).
